# EUROPEAN PATENT APPLICATION

(11) **EP 2 347 758 A1**
(43) Date of publication of application: **27.07.2011**
(21) Application number: 09825024.4
(22) Date of filing: 29.07.2009
(51) Int. Cl.: A61K 31/505, A61K 9/28

(54) **PROCESS FOR OBTAINING A COMPOSITION OF ROSUVASTATIN CALCIUM AND PRODUCT OBTAINED**

(30) Priority: 10.11.2008 MX 2008014321
(71) Applicant: Psicofarma S.a. De C.v., México D.F. (MX)
(72) Inventor: MAYA AYALA, Kenia Lizeth, México D. F. (MX); ESTRADA FLORES, Luis, México D. F. (MX)
(74) Representative: Carvajal y Urquijo, Isabel
(86) International application number: PCT/MX2009/000081
(87) International publication number: WO 2010/053343

(57) **Abstract**

The present invention describes a rosuvastatin calcium composition that does not require tribasic phosphate salts to be stable and that also has suitable bioavailability, and is helpful in reducing lipid and/or cholesterol levels in the body, as well as the manufacturing method of this composition.

## Description

### TECHNICAL FIELD OF THE INVENTION

The present invention is related to the field of pharmaceutical chemistry in the processes for obtaining medicine compositions, and in particular, the present invention refers to the preparation of a rosuvastatin calcium composition in film-coated tablet form for oral administration, and for application in the reduction of lipid and/or cholesterol levels in the body.

### BACKGROUND

Rosuvastatin is a member of the class of drugs known as statins, used to lower cholesterol. Rosuvastatin inhibits the enzyme responsible for changes that produce the mevalonate located in the fine hepatic tissue, a small molecule used in the synthesis of cholesterol and other mevalonate derivatives. This reduces the amount of cholesterol produced as well as alternately reducing the total amount of LDL cholesterol.

As with other statins, Rosuvastatin is a competitive inhibitor of HMG-CoA reductase, and a fully synthetic compound. HMG-CoA reductase catalyzes the reduction of 3-hydroxy-3-methylglutaryl-coenzyme A (HMG-CoA) to mevalonate, which is the rate-limiting step in hepatic biosynthesis of cholesterol. The inhibition of the enzyme reduces de novo cholesterol synthesis, increasing the expression of the low-density lipoprotein receptors (LDL receptors) in hepatocytes. This increases LDL in the hepatocytes, reducing the amount of LDL-cholesterol in the blood. Like other statins, Rosuvastatin also reduces blood triglyceride levels and slightly increases HDL-cholesterol levels. Rosuvastatin is also indicated as a diet complement for the treatment of dyslipidemia, specifically hypercholesterolemia.

It is known through pharmaceutical and patent literature that HMG-CoA reductase inhibitors and specifically statins are incredibly difficult to make in tablet form due to the fact that they are sensitive to the micro-atmosphere of the composition and specifically to factors such as light, heat and humidity. Pharmaceutical compositions containing Rosuvastatin, acid (3R,5S,6E)-7-[4-(4-Fluorophenyl)-6-(1-methylethyl)-2-[methyl(methylsulfonyl) amino] -5-pirimidinyl] - 3,5-dihydroxy-6-heptenoic, and their pharmaceutically acceptable salts were announced in the Mexican patent 215601, which corresponds to the North American patent US 6,316,460. This patent covers pharmaceutical compositions in its calcium and sodium salts, comprising acid (E) - 7- [4-(4-fluorophenyl) - 6 - isopropyl - 2 - [metil(metilsulfonil) amino] pyrimidin - 5 - yl] - (3R,5S) - 3,5 - dihydroxy hept - 6 enoic, which corresponds to rosuvastatin as an active ingredient and a tribasic phosphate salt with cation multivalent in ratios from 1:80 to 50:1 of tribasic phosphate salt / active ingredient comprising one or more fillers. The active ingredient is present in 1-80% of the composition weight, the phosphate salt in 1 to 50% of the composition weight, the filler in 30 to 90% of the weight, the agglutinant substance in 2 to 90%, the disintegrant in 2 to 10% and the lubricant in 0.5 to 3% of the weight. An example of the composition includes acid or a salt plus tribasic calcium phosphate, microcrystalline cellulose, lactose, sodium starch glycolate, butylated hydroxytoluene and magnesium stearate. Another composition example adds povidone and mannitol, and yet another composition has lactose. It also covers the use of tribasic phosphate salt in which the cation is multivalent to stabilize the acid compound. The phosphate salt is tribasic calcium phosphate, tribasic magnesium phosphate and tribasic aluminum phosphate. Lastly it covers the production method of said composition. Said method consists of the dry mix of the active ingredient, tribasic phosphate salt, the antioxidant and the rest of the excipients to be compressed into tablets; the main disadvantage of this method is the potential problems with the flow and uniformity of weight of the resulting tablets, as well as the possibility that the alkaline earth metal salts might irritate the intestinal mucosa. This formulation must be prepared in a package with a strong seal that protects it from humidity such as an Alu-alu blister pack, as otherwise the formulation does not remain stable enough to enter the market and be sold and used.

The Mexican patent 227360 announced a pharmaceutical composition comprising the HMG-CoA reductase inhibitor (E) - 7- [4-(4-fluorophenyl) - 6 - isopropyl - 2 - [metil(metilsulfonil) amino] pirimidin - 5 - il] - (3R,5S) - 3,5 - dihydroxy hept - 6 enoic or a similar pharmaceutically acceptable salt, which corresponds to rosuvastatin as an active ingredient and an inorganic salt in which the cation is multivalent. In this case the salt is aluminum magnesium metasilicate in ratios from 1:80 to 50:1 by weight of inorganic salt /active ingredient containing one or more fillers, agglutinants, disintegrants or lubricants. The active ingredient is present in 1-50% of the composition weight, the inorganic salt in between 1 and 50% of the composition weight, the filler in 30 to 90% of the weight, the agglutinant substance in 2 to 90%, the disintegrant in 2 to 10% and the lubricant in 0.5 to 3% of the weight. However, none of the composition examples included in this patent contain acid mixtures or a salt plus aluminum and magnesium metasilicate, but do exclusively mention the same compositions of patent no. 215601, that is, mixtures with tribasic calcium phosphate.

The patent WO 2008/035128 Al applied for by Gedeon Richter (Hungary) is related to a pharmaceutical composition that comprises rosuvastatin calcium as the active ingredient and magnesium hydroxide and/or calcium acetate or calcium gluconate or calcium glycerophsphate or aluminum hydroxide as stabilizing excipients and one or more acceptable pharmaceutical excipients such as lactose, microcrystalline cellulose, PVP, crospovidone and magnesium stearate. This patent also states that one of the main problems that have been experienced in the prior methods used is precisely in finding the best rosuvastatin calcium composition that shows the best stability characteristics, which is precisely the contribution it makes in this sense. Moreover it refers to various other inventions where the rosuvastatin calcium composition has variants with regard to components and forms of preparation.

From everything analyzed in the prior methods used it is considered that the problem of stability in the rosuvastatin calcium compositions has not been solved in any case, especially in both the addition of components that give it this characteristic of stability and also in its use as a pharmaceutical product in easy-to-use and reasonably-priced containers.

The present invention is related to a process to obtain a new rosuvastatin calcium composition (calcium salt from the acid (3R,5S,6E)-7-[4-(4-Fluorophenyl)-6-(1-methylethyl)-2-[methyl(methylsulfonyl) amino]-5-pyrimidinyl]-3,5-dihydroxy-6-heptenoic) in which, due to the way in which its preparation process is carried out, does not require tribasic phosphate salts to be stable or any other similar agent for this purpose, and that moreover said composition also has an appropriate bioavailability of the active ingredient. The process for obtaining of the present composition is new and highly effective and enables a significant advance to be made in the prior methods used.

### DETAILED DESCRIPTION OF THE INVENTION

The resultant composition of the object of the present invention allows a therapeutically effective amount of rosuvastatin to be supplied, which is helpful in the treatment and/or prevention of conditions that benefit from the reduction of lipid and/or cholesterol levels in the body. The object composition of the present invention can be used in solid forms of dosage such as powders, tablets or capsules and due to its degree of stability which distinguishes it from the other known compositions, it can be prepared in different types of packaging, and not only packs strongly sealed to protect them against humidity like Alu-alu blister packs. By the same token an advantage for its administration to patients is that due to its lack of alkaline earth metal salts, this composition is less irritating to the gastrointestinal tract.

The object composition of the present invention involves different types of excipients which enable the object to make the rosuvastatin bioavailable and which are generally used in the manufacture of tablets or pills, among other pharmaceutical forms. The materials known as excipients must comply with a series of physiochemical and rheological properties such as: porosity, particles density, flow property, compaction, and others.

Often, the single dose of the active component is small and the inert or dilution substance is added to increase the volume, so that the tablet is of a size that is practical for compression. Consequently, it is fundamental that the dilution shows appropriate compression characteristics, which will depend on numerous factors, such as crystallinity, water of crystallization and macro and microscopic structure. Many of the classic dilutions for tablets have today been modified to provide fluidity and compressibility, which allows them to have a plastic deformation in many cases like the size of granules that form during traditional moist granulation. Among these are lactose and microcrystalline cellulose.

Lactose is used as a dilution in formulations and exists in two isomeric forms: alpha lactose and beta lactose. Different kinds of lactose have been designed especially for direct compression, including: spray-dried lactose, lactose monohydrate and anhydrous lactose. Generally lactose is not affected by humidity and is not affected much by lubricants.

Another multi-functional excipient widely used in the pharmaceutical industry is the microcrystalline cellulose obtained through hydrolysis of cellulose fibers which produce a material capable of plastic deformation and that is therefore widely compressible.

Antioxidants perform a fundamental role guaranteeing that drugs such as rosuvastatin (susceptible to oxidation and hydrolysis with the formation of a lactone) maintain their activity, taste and color, and can be used for longer periods of time. Their use is especially helpful in avoiding the oxidation of the other elements and the products containing them. When the antioxidants are added to the pharmaceutical formulations, the start of the final stages of auto-oxidation is delayed. The most common antioxidants usually belong to the ascorbic acid group, sodium ascorbate, calcium ascorbate, ascorbyl palmitate, tocopherols, gallates, butylhydroxylansinol, butylhydroxytoluene, among others.

The disintegrants are substances or mixtures of substances that encourage in a pill its disintegration in a water environment, increasing its area and allowing the quick release of the active substance. The active substances must be released from the mold of the pill, as effectively as possible, breaking the unions formed during compression like the van der Waals forces, capillary unions, hydrogen bridges, fusion bonds or partial dissolution of areas with recrystallization. Within the action mechanism of a disintegrant are the following hypotheses: heat exchange produced during the hydration process, swelling, porosity, deformation and the breaking of physicochemical unions.

There are various substances that comply with this purpose, including sodium croscarmelose, which is a modified cellulose gum that helps pills to disintegrate and dissolve, which is effective in low-dose usage and with high levels of hardness. The fibrous nature of sodium croscarmelose provides it with excellent potential for water absorption and its reticulated chemical structure creates an insoluble hydrophilic product and high swelling properties. Sodium croscarmelose is effective when used in concentrations of 0.5% to 5.0%, producing tablets with excellent disintegration properties. The effectiveness of sodium croscarmelose can be reduced if a hygroscopic excipient is present in the tablet formulation.

Another of the disintegrants used is pregelatinized starch. This starch is soluble when cold, thickening when cold or tepid water is added, providing an excellent texture.

Lubricants perform various functions; the main one is to avoid the tablets sticking to the surface of .the punch and to reduce friction between the particles; the addition method is very important so that a lubricant fulfils its function. The problems that must be solved to produce an optimum lubrication are: an appropriate lubricant selection, concentration optimization, the appearance of possible collateral effects of lubrication, and the mixing time, type of mixer and the speed employed are also important. Magnesium stearate is a fine white low-density powder with a characteristic odor and color; the powder feels greasy and sticks to the skin. It is the most common and effective of the lubricants used in the formulation of pharmaceutical products. Magnesium stearate is generally effective at levels of 0.25% to 5.0%. It is mixed with the rest of the product for a short period of time (no more than 5 minutes) due to the adverse effects produced in the compaction and the problems it can cause in the dissolution. Magnesium stearate is incompatible with strong acids, alkalis and iron salts. Mixing it with antioxidant materials must be avoided.

Furthermore, the addition of a sliding agent can improve the flow of a granulation during unit compression, especially in the hoppers, the feeders and molds of a tableting machine, with which weight uniformity in the final tablet is assured. They also minimize the tendency of a granulation to separate or segregate because of excess vibration that could occur during compression. The best known sliding agents are talc, corn starch and colloidal silicon dioxide and other silicon and oxygen derivatives, that are normally used in proportions of 0.5-3.0% of the total weight of the tablet.

During the invention process it was determined that if the final pharmaceutical form is a tablet, it must be coated with a polymer mixture that acts as additional protection against humidity and oxidation with the purpose of providing extra protection for the active constituent. A tablet coating mixture must contain a film-forming polymer derived from cellulose or methacrylic acid such as hydroxypropyl cellulose (HPC), hydroxypropyl methylcellulose (HPMC), Ethyl cellulose (EC) or sodium methyl methacrylate; from an opacifying agent such as titanium dioxide which partially protects the product from light; and from a plastifying agent to reduce the vitreous transition temperature of the polymer and assure its flexibility, like triacetin, stearic acid or various degrees of polyethylene glycol; all this suspended in an appropriate solvent to form a suspension. The tendency is for the solvent to be aqueous. Additional ingredients such as lacquer colorants, glossy compounds or speed release modifiers allow organoleptic and/or functional changes to be made to the film coating.

During the invention process it was discovered that if the coating includes aluminum and magnesium silicon-based pearl pigments (Candurin®, Merck), the stability of the entire formula improves given that these pigments cause part of the light that influences the tablet to be refracted and subsequently reflected from the area (avoiding its penetration to the core of the tablet), and reduce the speed of water and oxygen transmission through the film; these actions maintain the integrity of rosuvastatin, as the coating of the product in tablet form is considered essential in the formulation, as the stability of the coated product was greater than that of the uncoated core.

During the invention process it was also found that the granulated manufacturing method is essential for the integrity and stability of the finished product. If the process is carried out differently to the one described in this patent the result is a mass that has the consistency of chewing gum where the active constituent is easily oxidized, especially when it is moistened during the manufacturing process, as this causes hydrolysis and oxidation. It was found that adding Rosuvastatin in dry form and the close contact between the active constituent and antioxidants like Butylhydroxyanisole y Butylhydroxytoluene, which have already been mentioned, were enough to eliminate the formation of free radicals that are a precursor of the entire oxidation process, and therefore obtain acceptable stability in the active constituent in the core.

Lastly it was found that the object composition of the present invention in its form of a core with a pearl coating is sufficiently stable to be able to be prepared in packaging less hermetically sealed than the Aluminum-aluminum blister pack, which translates into a significant cost reduction.

The object composition of the present invention comprises rosuvastatin calcium equivalent to approximately 5 to 10% of the weight of the active constituent; 0.01 to 0.02% of the weight of an antioxidant agent; 60 to 75% of the weight of a dilution or dilution mixture; 5 to 13% of the weight of one or more disintegrants, 0.5 to 5% of the weight of a sliding agent and 0.55 to 5% of the weight of one or several lubricants. This part of the composition can be in granulated form or in the form of an uncoated tablet.

The process that accompanies the object composition of the present invention and which is an intrinsic part of the composition consists in sifting through a mesh with an opening of between 590 and 840 micrometers some of the dilutions and the disintegrants; mixing with equipment suitable for dry mixing for 5-20 minutes; granulate this mixture with a solution of the chosen antioxidant in a mixture of Ethanol at 95% and water in proportions that go from 1:1 to 2:1; dry the granulated mixture at a temperature of between 40 - 75°C until between 1-3% humidity is obtained; sift the resulting granulated mixture through a mesh with openings of between 1,000 and 1,410 micrometers; mix the resulting granulated mixture with the rest of the dilutions, disintegrants, the sliding agent and the active constituent for 5-20 minutes; sift the lubricants through a mesh with openings of between 420 and 590 micrometers; mix the granulation with the lubricants for 2-10 minutes; and lastly compress the resulting granulated mixture or fill sachets or capsules with the weight appropriate to the desired dose of rosuvastatin that can be between 10 and 80 mg.

When the product is formulated as a tablet the composition includes a pearl coating that consists of a powder or granulated mixture that contains 55-70% of the weight of a particle-forming polymer; 15-25% of one or more plastifiers; 3-5% of lacquer colorants, 0.5-1.5% of an tenso-active agent, and 0.5 - 1.5% of an glossy agent. This powder is suspended in water purified at concentrations that vary from 15-25% weight/volume, and is sprayed in a conventional tablet-coating machine with a normal or perforated pump and spray pistols, until the finished tablet's final weight has increased by 2-5%.

In particular the object composition of the present invention comprises rosuvastatin calcium equivalent to approximately 5 to 10% of the weight of Rosuvastatin; 0.01 to 0.02% of butylhydroxyanisole or butylhydroxytoluene; 20 to 40% of monohydrated lactose; 20 to 45% of microcrystalline cellulose; 5.5. to 7% of sodium croscarmelose; 12 to 15% of pregelatinized starch; 0.5 to 5% of a silicon-based sliding agent (Aerosil® 200, Cab-O-Sil® or Neusilin®); 3-5% of hydrogenated vegetable oil and 0.55 to 1% of magnesium stearate, in tablet form and coated with a material consisting of 55-70% of the weight of low viscosity HPMC; 5-10% of PEG 6000; 15-20% of stearic acid; 3-5% of lacquer colorants, 0.5-1.5% of a tenso-active agent, and 0.5 - 1.5% of a glossy agent.

The manufacturing process for this formulation and which is an intrinsic part of the invention consists in particular of sifting through a mesh with an opening of between 590 and 840 micrometers between 50 and 60% of the total amount of monohydrated lactose dilutions and microcrystalline cellulose as well as between 50 and 60% of the pregelatinized starch disintegrants and sodium croscarmelose; mixing with equipment suitable for dry mixing for 5-20 minutes; granulate this mixture with a solution of the chosen antioxidant (butylhydroxyanisole or butylhydroxytoluene) in a mixture of Ethanol to 95% and water in proportions that go from 1:1 to 2:1; dry the granulated mixture at a temperature of between 40 - 75°C until between 1-3% humidity is obtained; sift the resulting granulated mixture through a mesh with and opening of between 1,000 and 1,410 micrometers; mix the resulting granulated mixture with the rest of the dilutions, disintegrants, the sliding agent (Aerosil 200, Cab-O-Sil or Neusilin) and the rosuvastatin for 5-20 minutes; sift the hydrogenated vegetable oil lubricants and magnesium stearate through a mesh with an opening of between 420 and 590 micrometers; mix the granulation with the lubricants for 2-10 minutes; and lastly compress the resulting granulated mixture or fill sachets or capsules with the weight appropriate to the desired dose of rosuvastatin which can be between 10 and 80 mg. The tablets are coated with the commercial coating Easy Pearlcoat ® Orange 401 (Nutrer, S.A. de C.V) which contains 55-70% of the weight of low viscosity HPMC; 5-10% of PEG 6000; 15-20% of stearic acid; 1-2% of lacquer colorant Yellow No.6; 1-2% of lacquer colorant Red No. 40, 0.5-1.5% of sodium lauryl sulfate; 2-5% of titanium dioxide and 0.5 - 1.5% of mica (Candurin®). This powder is suspended in water purified to concentrations that vary from 10-25% weight/volume and is sprayed in a conventional tablet-coating machine with a normal or perforated pump and spray pistols, until the weight of the finished tablet increases by 2-5%.

### Example

The following composition is a non-limitative example of the object compositions of the present invention: 41.6 mg of Rosuvastatin calcium (equivalent to 40 mg of Rosuvastatin); 0.070 mg of butylhydroxyanisole; 150 mg of monohydrated lactose; 240 mg of microcrystalline cellulose with a particle size close to 50-90 µm; 35 mg of sodium croscarmelose; 75 mg of pregelatinized starch; 25 mg of Aerosil 200; 20 mg of hydrogenated vegetable oil and 4.5 mg of magnesium stearate. The manufacturing process consists of sifting through a mesh with an opening of between 590 and 840 micrometers 50% of the total amount of the monohydrated lactose dilutions and microcrystalline cellulose and 60% of the pregelatinized starch disintegrants and sodium croscarmelose; mix with equipment suitable for dry mixing for 5-20 minutes; granulate this mixture with a solution of the antioxidant in a mixture of Ethanol to 95% and water in a proportion of 1:1; dry the granulated mixture at a temperature of 50°C until between 1-3% humidity is obtained; sift the resulting granulated mixture through a mesh with an opening of between 1,000 and 1,410 micrometers; mix the resulting granulated mixture with the rest of the dilutions, disintegrants, Aerosil 200 and the rosuvastatin for 5-20 minutes; sift the hydrogenated vegetable oil lubricants and magnesium stearate through a mesh with an opening of between 420 and 590 micrometers; mix the granulation with the lubricants for 2-10 minutes; and lastly compress the resulting granulated mixture to a weight of 591mg ±5%. The tablets are coated with the commercial coating Easy Pearlcoat Orange 401 (Nutrer, S.A. de C.V) suspended in water to 15% and sprayed in a conventional tablet-coating machine with a normal pump and spray pistols, until the weight of the finished tablet increases by 4%.

The tablets resulting from the example composition have been prepared in bubble wrap (blister pack) which consists of a Polyvinyl Chloride and Polyvinylidene Chloride (PVC/PVDC) film between 254 and 304 micrometers thick, thermoformed to create small receptacle containers for the tablet, and covered with a 25-micrometer thick aluminum sheet. The packaging obtained by combining both components is known as "PVC/PVDC/Aluminum blister."

The same formulation was also prepared in similar wrapping comprising two sheets of a multilaminate consisting of a 25-micron thick polyamide film, bound using a polyurethane-based lacquer to a 45-micrometer thick aluminum film bound in turn with the same adhesive to a 60-micrometer thick PVC laminate. Two identical laminates, one of which undergoes a cold process to create small receptacle containers for the tablet, are bound together through heat to form a form of packaging known as "Alu-alu blister" and which is the packaging used by the commercial formulation of Rosuvastatin protected by patents 215601 and 227360, characterized by its impenetrability by both oxygen and humidity.

The tablets from the example formulation, prepared in both types of packaging were submitted to extreme humidity and temperature conditions (40°C ± 0.5°C, 75% ± 5% Relative Humidity) following the NOM-073-SSA1-2005 of Stability of Medicine for a period of 3 months. The results of the trials on the active constituent and its main degradation products show that the example formulation is stable both in the PVC/PVDC/Aluminio blister and in the Alu-alu blister.

| **EVALUATIÓN FORMULA 10 mg** | | |
|---|---|---|
| **TIME(months)** | **Alu/PVDC** | **Alu/Alu** |
| 0 | 94.2426 | 95.3352 |
| 30 | 92.6036 | 96.1167 |
| 60 | 93.4632 | 96.2289 |
| 90 | 94.3730 | 95.6576 |

| **% DISSOLVED AT 30 MINUTES** | | |
|---|---|---|
| **TIME(months)** | **Alu/PVDC** | **Alu/Alu** |
| 0 | 99.6973 | 100.3981 |
| 30 | 98.4535 | 100.3076 |
| 60 | 97.2728 | 98.3234 |
| 90 | 96.0500 | 97.6017 |

In both cases the amount of related substances and degradation products (lactone and the oxidized substance) was "not detectable" to the quantification trial by HPLC. The dissolution remains at 40°C/75% H.R. for more than a year, which is equivalent to more than two years at room temperature.

## Claims

1. Rosuvastatin calcium composition, **characterized** because it contains rosuvastatin calcium equivalent to approximately 5 to 10% of the weight of the active constituent; 0.01 to 0.02% of the weight of an antioxidant agent; 60 to 75% of the weight of a dilution or dilution mixture; 5 to 13% of the weight of one or more disintegrants, 0.5 to 5% of the weight of a slipping agent and 0.55 to 5% o the weight of one or several lubricants.

2. The composition in accordance with justification 1, **characterized** because the antioxidant agent is selected from the group that consists of ascorbic acid, sodium ascorbate, calcium ascorbate, ascorbyl palmitate, tocopherols, gallates, butylhydroxylanisole, butylhydroxytoluene, among others

3. The composition in accordance with justification 1, **characterized** because the dilution or inert agents are selected from the group that consists of spray dried lactose, monohydrated lactose, anhydrous lactose and microcrystalline cellulose.

4. The composition in accordance with justification 1, **characterized** because the disintegrant agents are selected from the group that consists of starch, pregelatinized starch, sodium croscarmelose, sodium glycolate and starch.

5. The composition in accordance with justification 1, **characterized** because the slipping agent is selected from the group that consists of talc, corn starch and colloidal silicon dioxide and other silicon and oxygen derivatives.

6. The composition in accordance with justification 1, **characterized** because the lubricating agents are selected from the group that consists of stearic acid, magnesium stearate and hydrogenated vegetable oil.

7. The composition in accordance with justification 1, **characterized** because it contains rosuvastatin calcium equivalent to approximately 5 to 10% of the weight of Rosuvastatin; 0.01 to 0.02% of butylhydroxyanisole or butylhydroxytoluene; 20 to 40% of monohydrated lactose; 20 to 45% of microcrystalline cellulose; 5.5. to 7% of sodium croscarmelose; 12 to 15% of pregelatinized starch; 0.5 to 5% of a silicon-based slipping agent (Aerosil 200, Cab-O-Sil or Neusilin); 3-5% of hydrogenated vegetable oil and 0.55 to 1% of magnesium stearate.

8. The composition in accordance with justification 1, **characterized** because it is manufactured in accordance with the following procedure: sift through a mesh with an opening of between 590 and 840 micrometers some of the dilutions and disintegrants; mix with equipment suitable for dry mixing for 5-20 minutes; granulate this mix with a solution of the chosen antioxidant in a mixture of Ethanol to 95% and water in proportions that go from 1:1 to 2:1; dry the granulated mixture at a temperature of between 40 - 75°C until between 1-3% humidity is obtained; sift the resulting granulated mixture through a mesh with an opening of between 1,000 and 1,410 micrometers; mix the resulting granulated mixture with the rest of the dilutions, disintegrants, the slipping agent and the active constituent for 5-20 minutes; sift the lubricants through a mesh with an opening of between 420 and 590 micrometers; mix the granulation with the lubricants during 2-10 minutes; and lastly compress the resulting granulated mixture or fill sachets or capsules with the weight appropriate to the desired dose of rosuvastatin which can be between 10 and 80 mg.

9. The composition in accordance with justification 1, **characterized** because it is manufactured in accordance with the following procedure: sift through a mesh with an opening of between 590 and 840 micrometers between 50 and 60% of the total amount of the monohydrated lactose dilutions and microcrystalline cellulose as well as between 50 and 60% of the pregelatinized starch disintegrants and sodium croscarmelose; mix with equipment suitable for dry mixing for 5-20 minutes; granulate this mixture with a solution of the chosen antioxidant (either butylhydroxyanisole or butylhydroxytoluene) in a mixture of Ethanol to 95% and water in proportions that go from 1:1 to 2:1; dry the granulated mixture at a temperature of between 40 - 75°C until between 1-3% humidity is obtained; sift the resulting granulated mixture through a mesh with an opening of between 1,000 and 1,410 micrometers; mix the resulting granulated mixture with the rest of the dilutions, disintegrants, the slipping agent (Aerosil 200, Cab-O-Sil or Neusilin) and the rosuvastatin for 5-20 minutes; sift the hydrogenated vegetable oil lubricants and magnesium stearate through a mesh with an opening of between 420 and 590 micrometers; mix the granulation with the lubricants for 2-10 minutos; and lastly compress the resulting granulated mixture or fill sachets or capsules with the weight appropriate to the desired dose of rosuvastatin which can be between 10 and 80 mg.

10. The composition in accordance with justification 1, **characterized** because after being compressed into tablets, the tablets are coated with a pearl coating which consists of a powder or granulated mixture which contains 55-70% of the weight of a particle-forming polymer; 15-25% of one or more plastifiers; 3-5% of lacquer colorants, 0.5-1.5% of a tenso-active agent, and 0.5 - 1.5% of a glossy agent. This powder is suspended in water purified at concentrations that vary from 15-25% weight/volume.

11. The composition in accordance with justifications 1 and 10, **characterized** because the coating is Easy Pearlcoat® Orange 401 (Nutrer, S.A. de C.V) which contains 55-70% of the weight of low viscosity HPMC; 5-10% of PEG 6000; 15-20% of stearic acid; 1-2% of lacquer colorant Yellow No.6; 1-2% of lacquer colorant Red No. 40, 0.5-1.5% of sodium lauryl sulfate; 2-5% of titanium dioxide and 0.5 - 1.5% of mica (Candurin ®). This powder is suspended in water purified at concentrations that vary from 10-25% weight/volume and are sprayed in a conventional tablet-coating machine with a normal or perforated pump and spray pistols, until the weight of the finished tablet increases by 2-5%.

12. Use of the rosuvastatin calcium composition in accordance with justification 1, for the treatment and/or prevention of conditions that benefit from the reduction of lipid and/or cholesterol levels in the body.

## Amended claims

### Amended claims under Art. 19.1 PCT

**1.** Rosuvastatin calcium composition, **characterized** because it comprise rosuvastatin calcium equivalent to 5 to 10% of the weight of the active constituent; 0.01 to 0.02% of the weight of an antioxidant agent; 60 to 75% of the weight of a dilution or dilution mixture; 5 to 13% of the weight of one or more disintegrants, 0.5 to 5% of the weight of a slipping agent and 0.55 to 5% of the weight of one or several lubricants.

**2.** The composition in accordance with justification 1, **characterized** because the antioxidant agent is selected from the group that consists of ascorbic acid, sodium ascorbate, calcium ascorbate, ascorbyl palmitate, tocopherols, gallates, butylhydroxylanisole, butylhydroxytoluene, among others

**3.** The composition in accordance with justification 1, **characterized** because the dilution or inert agents are selected from the group that consists of spray dried lactose, monohydrated lactose, anhydrous lactose and microcrystalline cellulose.

**4.** The composition in accordance with justification 1, **characterized** because the disintegrant agents are selected from the group that consists of starch, pregelatinized starch, sodium croscarmelose, sodium glycolate and starch.

**5.** The composition in accordance with justification 1, **characterized** because the slipping agent is selected from the group that consists of talc, corn starch and colloidal silicon dioxide and other silicon and oxygen derivatives.

**6.** The composition in accordance with justification 1, **characterized** because the lubricating agents are selected from the group that consists of stearic acid, magnesium stearate and hydrogenated vegetable oil.

**7.** The composition in accordance with justification 1, **characterized** because it comprise rosuvastatin calcium equivalent to 5 to 10% of the weight of Rosuvastatin; 0.01 to 0.02% of butylhydroxyanisole or butylhydroxytoluene; 20 to 40% of monohydrated lactose; 20 to 45% of microcrystalline cellulose; 5.5. to 7% of sodium croscarmelose; 12 to 15% of pregelatinized starch; 0.5 to 5% of a silicon-based slipping agent (Aerosil 200, Cab-O-Sil or Neusilin); 3-5% of hydrogenated vegetable oil and 0.55 to 1% of magnesium stearate.

**8.** The composition in accordance with justification 1, **characterized** because it is manufactured in accordance with the following procedure: sift through a mesh with an opening of between 590 and 840 micrometers some of the dilutions and disintegrants; mix with equipment suitable for dry mixing for 5-20 minutes; granulate this mix with a solution of the chosen antioxidant in a mixture of Ethanol to 95% and water in proportions that go from 1:1 to 2:1; dry the granulated mixture at a temperature of between 40 - 75°C until between 1-3% humidity is obtained; sift the resulting granulated mixture through a mesh with an opening of between 1,000 and 1,410 micrometers; mix the resulting granulated mixture with the rest of the dilutions, disintegrants, the slipping agent and the active constituent for 5-20 minutes; sift the lubricants through a mesh with an opening of between 420 and 590 micrometers; mix the granulation with the lubricants during 2-10 minutes; and lastly compress the resulting granulated mixture or fill sachets or capsules with the weight appropriate to the desired dose of rosuvastatin which can be between 10 and 80 mg.

**9.** The composition in accordance with justification 1, **characterized** because it is manufactured in accordance with the following procedure: sift through a mesh with an opening of between 590 and 840 micrometers between 50 and 60% of the total amount of the monohydrated lactose dilutions and microcrystalline cellulose as well as between 50 and 60% of the pregelatinized starch disintegrants and sodium croscarmelose; mix with equipment suitable for dry mixing for 5-20 minutes; granulate this mixture with a solution of the chosen antioxidant (either butylhydroxyanisole or butylhydroxytoluene) in a mixture of Ethanol to 95% and water in proportions that go from 1:1 to 2:1; dry the granulated mixture at a temperature of between 40 - 75°C until between 1-3% humidity is obtained; sift the resulting granulated mixture through a mesh with an opening of between 1,000 and 1,410 micrometers; mix the resulting granulated mixture with the rest of the dilutions, disintegrants, the slipping agent (Aerosil 200, Cab-O-Sil or Neusilin) and the rosuvastatin for 5-20 minutes; sift the hydrogenated vegetable oil lubricants and magnesium stearate through a mesh with an opening of between 420 and 590 micrometers; mix the granulation with the lubricants for 2-10 minutos; and lastly compress the resulting granulated mixture or fill sachets or capsules with the weight appropriate to the desired dose of rosuvastatin which can be between 10 and 80 mg.

**10.** The composition in accordance with justification 1, **characterized** because after being compressed into tablets, the tablets are coated with a pearl coating which consists of a powder or granulated mixture which contains 55-70% of the weight of a particle-forming polymer; 15-25% of one or more plastifiers; 3-5% of lacquer colorants, 0.5-1.5% of a tenso-active agent, and 0.5 - 1.5% of a glossy agent. This powder is suspended in water purified at concentrations that vary from 15-25% weight/volume.

**11.** The composition in accordance with justifications 1 and 10, **characterized** because the coating is Easy Pearleoat® Orange 401 (Nutrer, S.A. de C.V) which contains 55-70% of the weight of low viscosity HPMC; 5-10% of PEG 6000; 15-20% of stearic acid; 1-2% of lacquer colorant Yellow No.6; 1-2% of lacquer colorant Red No. 40, 0.5-1.5% of sodium lauryl sulfate; 2-5% of titanium dioxide and 0.5 - 1.5% of mica (Candurin ®). This powder is suspended in water purified at concentrations that vary from 10-25% weight/volume and are sprayed in a conventional tablet-coating machine with a normal or perforated pump and spray pistols, until the weight of the finished tablet increases by 2-5%.

**12.** Use of the rosuvastatin calcium composition in accordance, with justification 1, for the treatment and/or prevention of conditions that benefit from the reduction of lipid and/or cholesterol levels in the body.
